# EUROPEAN PATENT APPLICATION

(11) **EP 4 582 408 A1**
(43) Date of publication of application: **09.07.2025**
(21) Application number: 23860429.2
(22) Date of filing: 30.08.2023
(51) Int. Cl.: C07C 29/76, C07C 31/04, B01D 53/72, B01D 53/82, B01J 20/02, B01J 20/34, C02F 1/28

(54) **METHANOL ADSORBENT, METHOD FOR PRODUCING HIGH-CONCENTRATION METHANOL, AND METHANOL RECOVERY DEVICE**

(30) Priority: 02.09.2022 JP 2022139813
(71) Applicant: National Institute of Advanced Industrial Science and Technology, Chiyoda-ku Tokyo 100-8921 (JP)
(72) Inventor: SHUDO, Yuta, Tsukuba-shi, Ibaraki 305-8560 (JP); TAKAHASHI, Akira, Tsukuba-shi, Ibaraki 305-8560 (JP); KAWAMOTO, Tohru, Tsukuba-shi, Ibaraki 305-8560 (JP)
(74) Representative: Dehns
(86) International application number: PCT/JP2023/031589
(87) International publication number: WO 2024/048667

(57) **Abstract**

A methanol adsorbent for adsorbing methanol contained in a liquid or gas comprising water and methanol,
wherein the methanol adsorbent comprises, as an active component, a Prussian blue analog represented by the following general formula (1):

AₓM[M'(CN)₆]y·zH₂O (1)

(wherein x, y, and z satisfy 0 ≦ x ≦ 3, 0.1 ≦ y ≦ 1.5, and 0 ≦ z ≦ 6; A represents one or more of hydrogen ions, ammonium cations, alkali metal ions, and alkaline earth metal ions; and M and M' are cations independent of each other, excluding hydrogen ions, ammonium cations, alkali metal ions, and alkaline earth metal ions.)

## Description

### Technical Field

The present invention relates to a methanol adsorbent that adsorbs methanol from water or gas, as well as a method for producing high-concentration methanol and a methanol recovery device utilizing this methanol adsorbent.

### Background Art

Methanol, while harmful to humans, is also utilized as a resource. Its applications span a wide range, including as a synthetic raw material and solvent in the chemical industry, and as a fuel for direct combustion and fuel cells. Furthermore, methanol can be produced from raw materials such as biomass or CO₂, enabling its use as a carbon-neutral substance, with demand steadily increasing. If methanol contained in exhaust gas or wastewater could be recovered at high concentrations, it could serve as a valuable resource. For example, methanol with a concentration of 54 wt% or higher can be used in commercially available direct methanol fuel cells.

When utilizing methanol as a resource, it is important for the methanol concentration to be high. Wastewater, of course, and even exhaust gas contain water as water vapor, so separating methanol from water is important for recovering methanol at high concentrations. Prussian blue analogs, which are used as adsorbents for ammonia and cesium ions, are known to adsorb a large amount of methanol in environments without coexisting substances (Non-Patent Literature 1).

### Prior Art Literature

### Non-Patent Literature

[Non-Patent Literature 1] M. Asai, et al., J. Phys. Chem. C 2018, 122, 22, 11918-11925

### Summary of the Invention

### Technical Problem

However, Prussian blue analogs, such as those described in Non-Patent Literature 1, exhibit high affinity for water molecules in addition to methanol. Consequently, it remains unclear whether the Prussian blue analogs described in Non-Patent Literature 1 can adsorb methanol in water or in gas containing water vapor. Furthermore, even if these Prussian blue analogs can adsorb methanol, a significant problem exists in that it is not easy to selectively desorb methanol when water is also adsorbed simultaneously.

The present invention was made in view of such circumstances and aims to provide a methanol adsorbent capable of adsorbing methanol from liquids or gases containing water and methanol, as well as a method for producing high-concentration methanol and a methanol recovery device using this methanol adsorbent.

### Means for Solving the Problem

To solve the above problem, the following methanol adsorbent, method for producing high-concentration methanol, and methanol recovery device are provided.
[1] A methanol adsorbent for adsorbing methanol contained in a liquid or gas comprising water and methanol,
   wherein the methanol adsorbent comprises, as an active component, a Prussian blue analog represented by the following general formula (1):

      AₓM[M'(CN)₆]y·zH₂O (1)
   (wherein x, y, and z satisfy 0 ≦ x ≦ 3, 0.1 ≦ y ≦ 1.5, and 0 ≦ z ≦ 6; A represents one or more of hydrogen ions, ammonium cations, alkali metal ions, and alkaline earth metal ions; and M and M' are cations independent of each other, excluding hydrogen ions, ammonium cations, alkali metal ions, and alkaline earth metal ions.)
[2] The methanol adsorbent according to [1],
   wherein M is any one of Mn²⁺, Co²⁺, Ga²⁺, Ni²⁺, or Zn²⁺, and M' is Co³⁺ or Fe³⁺.
[3] The methanol adsorbent according to [1],
   wherein M is Mn²⁺, and M' is Co³⁺ or Fe³⁺.
[4] A method for producing high-concentration methanol, comprising:
   an adsorption step of bringing the methanol adsorbent according to any one of [1] to [3] into contact with a liquid or gas comprising water and methanol to adsorb water and methanol onto the methanol adsorbent;
   a water desorption step of maintaining the methanol adsorbent, after the adsorption step, at a first temperature to desorb water from the methanol adsorbent; and
   a methanol desorption step of maintaining the methanol adsorbent, after the water desorption step, at a second temperature higher than the first temperature to desorb and recover high-concentration methanol from the methanol adsorbent.
[5] The method for producing high-concentration methanol according to [4],
   wherein the first temperature is 70°C or higher and less than 120°C, and the second temperature is 120°C or higher and 250°C or lower.
[6] A method for producing high-concentration methanol, comprising:
   a methanol adsorption step of bringing the methanol adsorbent according to any one of [1] to [3] into contact with a liquid or gas comprising water and methanol at a third temperature to selectively adsorb methanol onto the methanol adsorbent over water; and
   a methanol recovery step of maintaining the methanol adsorbent, after the methanol adsorption step, at a fourth temperature higher than the third temperature to desorb and recover high-concentration methanol from the methanol adsorbent.
[7] The method for producing high-concentration methanol according to [6],
   wherein the third temperature is 35°C or higher and less than 120°C, and the fourth temperature is 120°C or higher and 250°C or lower.
[8] A methanol recovery device comprising:
   a methanol adsorbent according to any one of [1] to [3];
   a holding part for retaining the methanol adsorbent therein;
   a heating part capable of heating the methanol adsorbent in the holding part to a temperature of 35°C or higher and 250°C or lower;
   a first supply part for supplying a liquid or gas comprising water and methanol to the methanol adsorbent in the holding part; and
   a recovery part for recovering high-concentration methanol desorbed from the methanol adsorbent in the holding part.

### Effect of the Invention

The methanol adsorbent of the present invention contains a specific Prussian blue analog as an active component that adsorbs both water and methanol. This Prussian blue analog primarily desorbs water at a first temperature and primarily desorbs methanol at a second, higher temperature. Therefore, by subjecting the methanol adsorbent of the present invention, which has adsorbed water and methanol, to the first temperature to desorb water, followed by the second temperature to recover methanol, high-concentration methanol can be obtained.

Additionally, the methanol adsorbent of the present invention selectively adsorbs methanol over water within a specific temperature range. Therefore, according to the methanol adsorbent and the method for producing high-concentration methanol of the present invention, high-concentration methanol can be obtained by contacting the methanol adsorbent with water and methanol within the specified temperature range to allow methanol adsorption, and then recovering the methanol adsorbed on the methanol adsorbent at a higher temperature. Furthermore, according to the methanol recovery device utilizing the methanol adsorbent of the present invention, high-concentration methanol can be efficiently recovered.

### Brief Description of the Drawings

[Fig. 1] A figure shows a flow example of the method for producing high-concentration methanol according to the first embodiment.
[Fig. 2] A figure shows a schematic diagram of the methanol recovery device according to the embodiment ((a) during methanol adsorption, (b) during methanol desorption).
[Fig. 3] A figure shows Powder X-ray diffraction charts of Prussian blue analogs obtained in Production Examples 1 to 6.
[Fig. 4] A figure shows the methanol adsorption capacity of each sample (Example 1).
[Fig. 5] A figure shows the differential adsorption heat curve for methanol on activated carbon (Example 2).
[Fig. 6] A figure shows the differential adsorption heat curve for methanol on an Mn-Co analog (Example 2).
[Fig. 7] A figure shows the differential adsorption heat curve for water on an Mn-Co analog (Example 2).
[Fig. 8] A figure shows the TG-MS curve of activated carbon (Example 3).
[Fig. 9] A figure shows the TG-MS curve of an Mn-Co analog (Example 3).
[Fig. 10] A figure shows the TG-MS curve of a Co-Co analog (Example 3).
[Fig. 11] A figure shows the TG-MS curve of a Ga-Fe analog (Example 3).
[Fig. 12] A figure shows the TG-MS curve of an Ni-Fe analog (Example 3).
[Fig. 13] A figure shows the desorption amount of methanol from an Mn-Co analog (Example 6).
[Fig. 14] A figure shows the relationship between equilibrium methanol concentration and methanol adsorption capacity at equilibrium (Example 9).
[Fig. 15] A figure shows graph comparing methanol adsorption capacities at 1000 mg-methanol/L equilibrium (Example 10).
[Fig. 16] A figure shows graph showing the relationship between methanol-water flow rate per adsorbent and methanol concentration after column water flow.

### Modes for Carrying Out the Invention

An embodiment of the methanol adsorbent of the present invention is described below.

### <Methanol Adsorbent (Prussian Blue Analog)>

The methanol adsorbent is an adsorbent capable of adsorbing methanol contained in a liquid or gas comprising water and methanol. Typically, the methanol adsorbent can adsorb methanol contained in water or methanol present in a gas containing water vapor. It should be noted that the methanol adsorbent of this embodiment may adsorb water along with methanol.

The methanol adsorbent of this embodiment comprises, as an active component, a Prussian blue analog represented by the following general formula (1) (hereinafter sometimes referred to as "the Prussian blue analog of the embodiment"):

AₓM[M'(CN)₆]y·zH₂O (1)

In the examples described later, a compound represented by AₓM[M'(CN)₆]_{y}·zH₂O may be referred to as an "M-M' analog."

Here, x, y, and z satisfy 0 ≦ x ≦ 3, 0.1 ≦ y ≦ 1.5, and 0 ≦ z ≦ 6 (the same applies hereinafter). "A" represents one or more of hydrogen ions, ammonium cations, alkali metal ions, and alkaline earth metal ions (the same applies hereinafter). Furthermore, M and M' are independent cations (the same applies hereinafter). However, M and M' are not hydrogen ions, ammonium cations, alkali metal ions, or alkaline earth metal ions (the same applies hereinafter). Examples of M include Mn²⁺, Co²⁺, Ga²⁺, Ni²⁺, Zn²⁺, In²⁺, Fe²⁺, and Cu²⁺. Among these, M is preferably Mn²⁺, Co²⁺, Ga²⁺, Ni²⁺, or Zn²⁺ due to the robust cubic structure that can adsorb a large amount of adsorbates (e.g., methanol).

The methanol adsorbent of this embodiment may include the above-described Prussian blue analog and a carrier supporting the Prussian blue analog. Examples of the carrier include zeolite, bentonite, and activated carbon. The methanol adsorbent may take the form of a powder of the Prussian blue analog of this embodiment or may be in the form of granules obtained by kneading the Prussian blue analog of this embodiment with a crosslinking agent. Alternatively, the methanol adsorbent may also take the form of the Prussian blue analog of this embodiment supported on a column.

The inventors have discovered that the methanol adsorption properties of Prussian blue analogs are influenced not only by the type of coordination-unsaturated M but also by the valence of M'. The Prussian blue analog of this embodiment possesses adsorption sites where the adsorption energy with methanol is higher than that with water. The valence of M' is preferably +3 or higher. Examples of such M' include Co³⁺, Fe³⁺, Mn³⁺, and Cr³⁺. Among these, M' is preferably Co³⁺ or Fe³⁺. From the perspective of high methanol adsorption capacity and clear separation of desorption temperatures between water and methanol, it is preferable that M is Mn²⁺.

Methanol binds more strongly than water to the Prussian blue analog of this embodiment. Therefore, the temperature at which methanol desorbs from the Prussian blue analog of this embodiment is higher than the temperature at which water desorbs. Consequently, by separating the desorption of water and methanol through a water desorption step at a relatively low temperature and a methanol desorption step at a relatively high temperature, high-concentration methanol can be recovered after the methanol desorption step. Specific examples of the Prussian blue analog of this embodiment include Mn[Fe(CN)₆]_{y}·zH₂O, Co[Cr(CN)₆]_{y}·zH₂O, Mn[Cr(CN)₆]_{y}·zH₂O, and Cr[Co(CN)₆]_{y}·zH₂O.

Next, an embodiment of the method for producing high-concentration methanol according to the present invention (First Embodiment, Second Embodiment) will be described.

### <First Embodiment of the Method for Producing High-Concentration Methanol>

The method for producing high-concentration methanol according to the first embodiment comprises an adsorption step, a water desorption step, and a methanol desorption step. In the present invention, high-concentration methanol preferably has a "methanol mass / (methanol mass + water mass) × 100" of 40% or higher, more preferably 60% or higher, even more preferably 80% or higher, and particularly preferably 90% or higher.

The following describes each step of the method for producing high-concentration methanol according to the first embodiment.

### (Adsorption Step)

In the adsorption step, the methanol adsorbent of the present invention is brought into contact with a liquid or gas comprising water and methanol, allowing water and methanol to be adsorbed onto the methanol adsorbent. The method of contact is not particularly limited and may include a batch method, in which the methanol adsorbent is mixed with the liquid or gas containing water and methanol in a container, or a column method, in which the material containing water and methanol is passed through the methanol adsorbent. It is believed that water is adsorbed within the lattice spaces of the Prussian blue analog in the methanol adsorbent, and methanol is adsorbed at the M' metal defect sites.

Examples of liquids comprising water and methanol include methanol aqueous solutions. Examples of gases comprising water and methanol include gases containing water vapor and methanol vapor. As long as it does not hinder the adsorption of water and methanol onto the methanol adsorbent, or the desorption of water and methanol from the methanol adsorbent at different temperatures, other substances may be included in the liquid or gas comprising water and methanol. Specifically, the liquid comprising water and methanol may contain various substances, such as NaCl, KCI, or CaCl₂. Examples of such liquids include wastewater from pulp factories or methanol aqueous solutions generated during the synthesis of methanol from CO₂. The gas comprising water and methanol may also include, for example, helium, argon, nitrogen, oxygen, or carbon dioxide. Examples of such gases include volatile gases from black liquor in paper mills or exhaust gases from chemical plants.

From the perspective of increasing the adsorption amount of methanol by the methanol adsorbent, the temperature at which the methanol adsorbent is brought into contact with the methanol aqueous solution (in other words, the temperature at which the Prussian blue analog of this embodiment adsorbs water and methanol) is preferably 60°C or lower. The lower limit of this temperature is the temperature at which the methanol aqueous solution does not freeze. The freezing temperature of the methanol aqueous solution depends on its methanol concentration but is generally 0°C or higher.

From the perspective of increasing the adsorption amount of methanol by the methanol adsorbent, the temperature at which the methanol adsorbent is brought into contact with a gas containing water vapor and methanol (in other words, the temperature at which the Prussian blue analog of this embodiment adsorbs water and methanol from the gas) is also preferably 60°C or lower. The lower limit of this temperature is a level that does not cause issues such as the formation of ice in the gas. While this depends on the concentration of salts or molecules dissolved in water, the temperature for adsorbing water and methanol from the gas is preferably, for example, -30°C or higher.

### (Water Desorption Step)

In the water desorption step, the methanol adsorbent of this embodiment, after undergoing the adsorption step, is maintained at a first temperature to desorb water from the methanol adsorbent. The first temperature is a temperature at which a large amount of water desorbs from the methanol adsorbent of this embodiment that has adsorbed both water and methanol. In the water desorption step, while a large amount of water is desorbed, a portion of the methanol may also be desorbed. From the perspective of desorbing a large amount of water and a small amount of methanol from the methanol adsorbent of this embodiment, the first temperature is preferably 70°C or higher and less than 120°C. It should be noted that maintaining the first temperature does not necessarily mean keeping the temperature fixed; the temperature may vary within the range where a large amount of water desorbs from the methanol adsorbent of this embodiment.

### (Methanol Desorption Step)

In the methanol desorption step, the methanol adsorbent of this embodiment, after undergoing the water desorption step, is maintained at a second temperature higher than the first temperature to desorb and recover high-concentration methanol from the methanol adsorbent. The second temperature is a temperature at which a large amount of methanol desorbs from the methanol adsorbent of this embodiment, which still retains a small amount of water that was not desorbed during the water desorption step. In the methanol desorption step, while a large amount of methanol is desorbed from the methanol adsorbent of this embodiment, a small amount of water that remains adsorbed after the water desorption step may also desorb. From the perspective of desorbing a large amount of methanol from the methanol adsorbent of this embodiment, the second temperature is preferably 120°C or higher and 250°C or lower.
It should be noted that maintaining the second temperature does not necessarily mean keeping the temperature fixed; the temperature may vary within the range where a large amount of methanol desorbs from the methanol adsorbent of this embodiment.

In the method for producing high-concentration methanol of this embodiment, the Prussian blue analog contained in the methanol adsorbent of this embodiment desorbs primarily water at the first temperature and primarily methanol at the second temperature, which is higher than the first temperature. Therefore, by desorbing water from the methanol adsorbent that has adsorbed both water and methanol at the first temperature, followed by desorbing methanol at the second temperature, high-concentration methanol can be recovered.

In the method for producing high-concentration methanol of this embodiment, recovering high-concentration methanol includes cooling a gas containing water vapor and methanol vapor to obtain a methanol aqueous solution with a high methanol content, as well as sending a gas containing water vapor and a greater amount of methanol vapor to the subsequent step following the methanol desorption process.

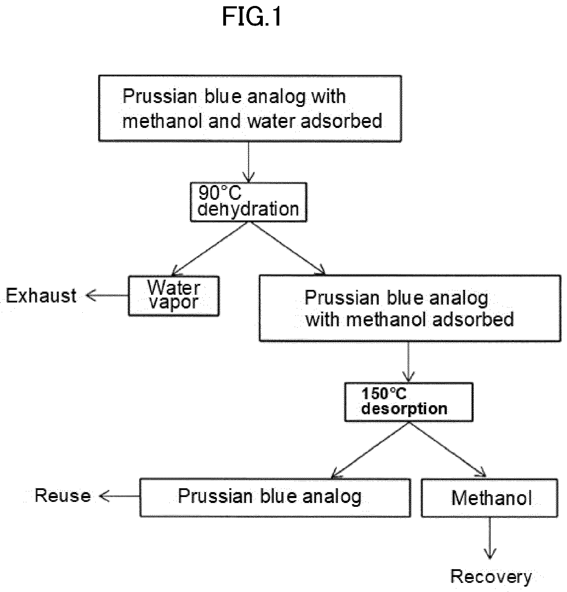

The method for producing high-concentration methanol according to the first embodiment is typically carried out using the flow shown in Figure 1. First, through the adsorption step, a Prussian blue analog that has adsorbed methanol and water is obtained. Next, this Prussian blue analog is heated to a first temperature of 90°C for dehydration (water desorption step). Through heating at 90°C, water vapor is desorbed from the Prussian blue analog. The desorbed water vapor is then exhausted.

On the other hand, after dehydration, methanol remains adsorbed on the Prussian blue analog. This Prussian blue analog is then heated to a second temperature of 150°C to desorb methanol (methanol desorption step). By heating at 150°C, the Prussian blue analog, from which water and methanol have been desorbed, is obtained along with high-concentration methanol, which may contain a small amount of water. This Prussian blue analog can be reused. In other words, it can be employed again in the adsorption step. Meanwhile, the high-concentration methanol desorbed from the Prussian blue analog can be recovered using an appropriate method.

### <Second Embodiment of the Method for Producing High-Concentration Methanol>

The method for producing high-concentration methanol according to the second embodiment includes a methanol adsorption step and a methanol recovery step. In the second embodiment, explanations of aspects common to the method for producing high-concentration methanol in the first embodiment (e.g., methods of contact, high-concentration methanol, and its recovery) are partially omitted. The inventors have discovered a novel finding that at a specific temperature (third temperature), when water and methanol are brought into contact with the methanol adsorbent of this embodiment, water is less likely to be adsorbed, while methanol is more easily adsorbed onto the methanol adsorbent. The second embodiment utilizes this finding.

### (Methanol Adsorption Step)

In the methanol adsorption step, the methanol adsorbent of the above-described embodiment is brought into contact with a liquid or gas comprising water and methanol at a third temperature, selectively adsorbing methanol onto the methanol adsorbent over water. As in the first embodiment, examples of the liquid or gas comprising water and methanol include methanol aqueous solutions or gases containing water vapor and methanol vapor.

As long as it does not hinder the adsorption of methanol onto the methanol adsorbent of this embodiment or the desorption of methanol from the methanol adsorbent, the liquid or gas comprising water and methanol may contain other substances.

In the methanol adsorption step, "selectively adsorbing methanol onto the methanol adsorbent over water" refers to a condition where the ratio of the mass of methanol adsorbed onto the methanol adsorbent to the mass of water adsorbed onto the methanol adsorbent (hereinafter sometimes referred to as the "selectivity ratio") is 2 or higher.

It should be noted that even in cases where water is not adsorbed onto the methanol adsorbent, this is included in the selective adsorption of methanol onto the methanol adsorbent over water. The ratio of the mass of methanol adsorbed onto the methanol adsorbent to the mass of water adsorbed onto the methanol adsorbent (selectivity ratio) is preferably 3 or higher, more preferably 5 or higher, and even more preferably 10 or higher.

The third temperature is the temperature at which methanol is adsorbed onto the methanol adsorbent of this embodiment with a high selectivity ratio. From the perspective of adsorbing methanol onto the methanol adsorbent with a high selectivity ratio, the third temperature is preferably 35°C or higher and less than 120°C. However, this lower temperature limit corresponds to the temperature required to produce a methanol aqueous solution with a concentration of 54 wt%, which is suitable for use as fuel for direct methanol fuel cells. If methanol aqueous solutions with a lower methanol concentration are available, the third temperature may be less than 35°C.

### (Methanol Recovery Step)

In the methanol recovery step, the methanol adsorbent of this embodiment, after undergoing the adsorption step, is maintained at a fourth temperature higher than the third temperature to desorb and recover high-concentration methanol from the methanol adsorbent. By this methanol recovery step, high-concentration methanol can be recovered from the methanol adsorbent, where methanol is adsorbed with a high selectivity ratio. From the perspective of efficiently desorbing methanol from the methanol adsorbent, the fourth temperature is preferably 120°C or higher and 250°C or lower.

Next, an embodiment of the methanol recovery device according to the present invention will be described.

### <Methanol Recovery Device>

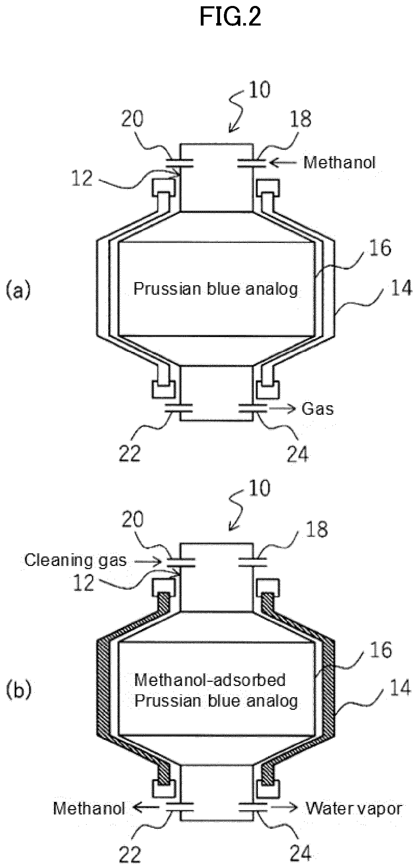

Figure 2 schematically illustrates an embodiment of the methanol recovery device 10 of the present invention. Figure 2(a) shows the methanol recovery device 10 during methanol adsorption, while Figure 2(b) shows the methanol recovery device 10 during methanol desorption. It should be noted that the methanol recovery device 10 is merely an example, and its configuration can be appropriately modified as long as the functional components of the methanol recovery device 10 perform their intended functions.

The methanol recovery device 10 comprises a methanol adsorbent (Prussian blue analog), a housing 12, and a heating part 14. The housing 12 includes a holding part 16, a first supply part 18, a second supply part 20, a recovery part 22, and a discharge part 24.

The holding part 16 has an internal void space in which the methanol adsorbent (Prussian blue analog) is retained. It is designed so that gases and liquids supplied to the holding part can pass through the methanol adsorbent (Prussian blue analog). The holding part 16 is configured to prevent the methanol adsorbent, as well as the passing gases and liquids, from leaking to the outside. The material of the holding part 16 is not particularly limited as long as it is corrosion-resistant to methanol and remains stable without degradation or deformation at the various temperatures involved in the production of high-concentration methanol. Examples of materials for the holding part 16 include stainless steel and heat-resistant fluororesins such as PTFE.

In the methanol recovery device 10 of this embodiment, the methanol adsorbent is the Prussian blue analog of this embodiment, and granular Prussian blue analog is placed inside the holding part 16, sandwiched between glass filters at the top and bottom. It should be noted that the form of the Prussian blue analog is not particularly limited as long as it allows for gas and liquid flow, does not undergo deformation, combustion, or degradation during the various processes for producing high-concentration methanol, and retains its methanol adsorption and desorption functionality. The Prussian blue analog may also be compounded with other materials.

For example, granular methanol adsorbents that include Prussian blue analog and other materials such as binders can effectively perform the function of adsorbing methanol. Additionally, the Prussian blue analog can be supported on other substrates. Examples of such substrates include fibers, nonwoven fabrics, glass filters, and honeycomb structures. As long as no issues arise during the various processes for producing high-concentration methanol, the methanol adsorbent may also take the form of a powder or slurry.

The heating part 14 is provided around the holding part 16 to heat the methanol adsorbent (Prussian blue analog) inside the holding part 16 to a temperature of 35°C or higher and 250°C or lower. The heating part 14 also heats the housing 12, the methanol adsorbent, and the gas or liquid supplied to the housing 12. As long as the interior of the housing 12 can be heated and maintained at a specific temperature, the shape of the heating part 14 or the heating mechanism of the heating part 14 is not particularly limited.

In the methanol recovery device 10, the heating part 14 is arranged around the holding part 16, but it may also be installed inside the housing 12. Additionally, the heating part 14 may include a linear heating element wrapped around the housing 12. The material of the heating element is not particularly limited as long as it does not undergo degradation, dissolution, or deformation. For example, in cases where the heating part 14 utilizes an electrical heating mechanism, a nichrome wire coated with rubber can be used as the heating element. In cases where the heating part 14 employs a liquid circulation-based temperature control mechanism, materials such as stainless steel or heat-resistant rubber can be used for the liquid circulation pipes. These circulation pipes are made of materials that do not dissolve in the liquid flowing through them.

The temperature control mechanism of the heating part 14 may adopt methods such as using electric heating wires, circulating heated steam or other gases, or circulating liquids, and it is not particularly limited. Additionally, if necessary, the heating part 14 may be equipped with a cooling mechanism. For example, the heating part 14 may include a device capable of controlling the temperature of a liquid, such as a heat exchanger. In this case, a liquid at a predetermined temperature is circulated near the housing 12 to control the temperature of the housing 12, allowing both heating and cooling of the housing 12.

The first supply part 18 supplies a methanol aqueous solution or a gas containing water vapor and methanol to the methanol adsorbent (Prussian blue analog) inside the holding part 16. The second supply part 20 supplies a gas that does not contain water vapor or methanol to the Prussian blue analog inside the holding part 16.

The recovery part 22 recovers high-concentration methanol desorbed from the methanol adsorbent (Prussian blue analog) inside the holding part 16. The discharge part 24 discharges the liquid or gas remaining after water and methanol have been adsorbed onto the methanol adsorbent (Prussian blue analog).

The materials of the first supply part 18, second supply part 20, recovery part 22, and discharge part 24 are not particularly limited as long as they do not undergo degradation, dissolution, or deformation during use. Examples of suitable materials include stainless steel and heat-resistant plastics.

In other embodiments of the methanol recovery device 10, the first supply part 18 and the second supply part 20 may be shared. That is, different components of liquid or gas may be supplied from the same supply part (either the first supply part 18 or the second supply part 20). Additionally, the first supply part 18 or the second supply part 20 may also function as a recovery part.

For example, high-concentration methanol can be recovered using the methanol recovery device 10 by following the procedure below.
The following describes an example where high-concentration methanol is recovered from a gas containing water vapor and methanol, but a similar procedure can also be applied to recover high-concentration methanol from a methanol aqueous solution.

With the second supply part 20 and the recovery part 22 closed, and the first supply part 18 and the discharge part 24 opened, as shown in Figure 2(a), a gas containing water vapor and methanol is supplied into the holding part 16, where the methanol adsorbent (Prussian blue analog) is installed, through the first supply part 18. At this time, if necessary, the temperature of the Prussian blue analog is adjusted using the heating part 14.

When the gas supplied from the first supply part 18 passes through the methanol adsorbent (Prussian blue analog), methanol is adsorbed onto the methanol adsorbent. Depending on the temperature of the methanol adsorbent, water may also be adsorbed onto the methanol adsorbent during this process. For example, when the methanol adsorbent (Prussian blue analog) is at a third temperature of 70°C, water is minimally or hardly adsorbed onto the methanol adsorbent, and primarily methanol is adsorbed. In contrast, for instance, when the methanol adsorbent (Prussian blue analog) is at 25°C, both water and methanol are adsorbed onto the methanol adsorbent. Downstream of the methanol adsorbent (Prussian blue analog), gas from which methanol has been adsorbed and removed is discharged through the discharge part 24.

If water and methanol are adsorbed onto the Prussian blue analog, the Prussian blue analog is heated to a first temperature (preferably 70°C or higher and less than 120°C) using the heating part 14. The first supply part 18 and the recovery part 22 are closed, while the second supply part 20 and the discharge part 24 are opened. As shown in Figure 2(b), a cleaning gas (e.g., dry air) that does not contain water vapor or methanol is supplied into the holding part 16, where the methanol adsorbent (Prussian blue analog) is installed, via the second supply part 20. Downstream of the methanol adsorbent (Prussian blue analog), the cleaning gas containing water vapor is discharged through the discharge part 24. Next, while continuing to supply the cleaning gas through the second supply part 20, the temperature of the methanol adsorbent (Prussian blue analog) is increased using the heating part 14. When the methanol adsorbent reaches the second temperature (preferably 120°C or higher and 250°C or lower), the discharge part 24 is closed, and the recovery part 22 is opened. High-concentration methanol desorbed from the Prussian blue analog is then discharged from the recovery part 22 and collected.

On the other hand, if little or no water is adsorbed onto the methanol adsorbent (Prussian blue analog) and methanol is adsorbed, the methanol adsorbent is heated to a fourth temperature (preferably 120°C or higher and 250°C or lower) using the heating part 14. The first supply part 18 and the discharge part 24 are closed, while the second supply part 20 and the recovery part 22 are opened. As shown in Figure 2(b), a cleaning gas is supplied into the holding part 16, where the methanol adsorbent (Prussian blue analog) is installed, via the second supply part 20. High-concentration methanol desorbed from the methanol adsorbent (Prussian blue analog) is discharged from the recovery part 22 and collected. Once the recovery of high-concentration methanol is complete, the methanol adsorbent (Prussian blue analog) can be reused. By increasing the frequency of reuse, the methanol recovery device 10 can be made smaller than a distillation apparatus used to distill and recover methanol from methanol aqueous solutions.

The methanol adsorbent, the method for producing high-concentration methanol, and the methanol recovery device of the present invention are not limited to the embodiments described above.

### [Examples]

The methanol adsorbent and the method for producing high-concentration methanol of the present invention are described below with reference to examples. However, the present invention is not limited to the following examples.

The following Prussian blue analogs were produced (Production Examples 1 to 6).

### Production Example 1: Mn-Co Analog

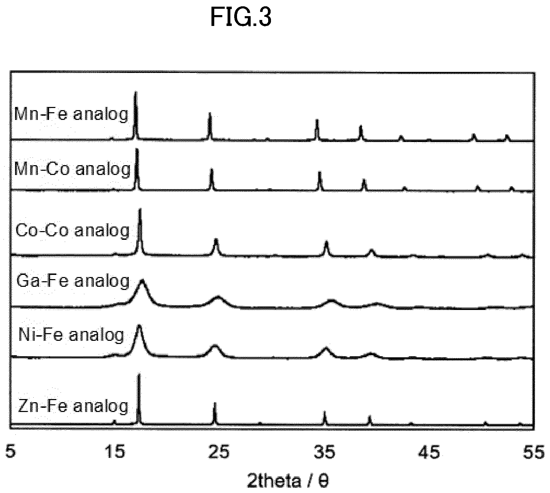

A solution of 20 mL of 0.6 mol/L manganese chloride aqueous solution was mixed with 20 mL of 0.3 mol/L potassium hexacyanocobaltate aqueous solution, shaken for 1 hour, and a precipitate was obtained. The liquid containing the precipitate was subjected to solid-liquid separation by centrifugation at 16,000 G for 10 minutes, and the supernatant was removed. After adding ultrapure water to the precipitate, the container was sealed and shaken vertically to agitate the precipitate in ultrapure water. This series of steps-centrifugation, removal of the supernatant, addition of ultrapure water, and agitation-was repeated six more times. Finally, the supernatant was removed, and the precipitate was vacuum dried at room temperature for 4 hours to obtain the Mn-Co analog powder. The powder X-ray diffraction pattern of this Mn-Co analog, as shown in Figure 3, was consistent with the diffraction pattern described in Reference A (X. Wu, et al., Cryst. Growth Des. 2006, 6, 26-28), confirming that the Mn-Co analog was K_{0.06}Mn[Co(CN)₆]_{0.66}·4.6H₂O.

### Production Example 2: Co-Co Analog

A Co-Co analog was obtained using the same method as in Production Example 1, except that the manganese chloride aqueous solution was replaced with a cobalt chloride aqueous solution. The powder X-ray diffraction pattern of this Co-Co analog, as shown in Figure 3, was consistent with the diffraction pattern described in Reference A, confirming that the Co-Co analog was KₓCo[Co(CN)₆]_{y}·zH₂O.

### Production Example 3: Ga-Fe Analog

A Ga-Fe analog was obtained using the same method as in Production Example 1, except that the manganese chloride aqueous solution was replaced with a gallium chloride aqueous solution, and the potassium hexacyanocobaltate aqueous solution was replaced with a sodium hexacyanoferrate aqueous solution. The powder X-ray diffraction pattern of this Ga-Fe analog, as shown in Figure 3, was consistent with the diffraction pattern described in Reference A, confirming that the Ga-Fe analog was KₓGa[Fe(CN)₆]_{y}·zH₂O.

### Production Example 4: Ni-Fe Analog

A Ni-Fe analog was obtained using the same method as in Production Example 1, except that the manganese chloride aqueous solution was replaced with a nickel nitrate aqueous solution, and the potassium hexacyanocobaltate aqueous solution was replaced with a potassium hexacyanoferrate aqueous solution.
The powder X-ray diffraction pattern of this Ni-Fe analog, as shown in Figure 3, was consistent with the diffraction pattern described in Reference A, confirming that the Ni-Fe analog was K_{0.01}Ni[Fe(CN)₆]_{0.69}·5.0H₂O.

### Production Example 5: Zn-Fe Analog

A Zn-Fe analog was obtained using the same method as in Production Example 1, except that the manganese chloride aqueous solution was replaced with a zinc sulfate aqueous solution, and the potassium hexacyanocobaltate aqueous solution was replaced with a potassium hexacyanoferrate aqueous solution. The powder X-ray diffraction pattern of this Zn-Fe analog, as shown in Figure 3, was consistent with the diffraction pattern described in Reference A, confirming that the Zn-Fe analog was KₓZn[Fe(CN)₆]_{y}·zH₂O.

### Production Example 6: Mn-Fe Analog

An Mn-Fe analog was obtained using the same method as in Production Example 1, except that the potassium hexacyanocobaltate aqueous solution was replaced with a sodium hexacyanoferrate aqueous solution. The powder X-ray diffraction pattern of this Mn-Fe analog, as shown in Figure 3, was consistent with the diffraction pattern described in Reference A, confirming that the Mn-Fe analog was K_{0.06}Mn[Fe(CN)₆]_{0.69}·4.5H₂O.

### Example 1: Methanol Adsorption Capacity Evaluation

The methanol adsorption capacity in a gas containing water and methanol was evaluated for six types of samples, including the Prussian blue analogs from Production Examples 1 to 5 and activated carbon (FUJIFILM Wako Pure Chemical Corporation, 037-02115, hereinafter the same). Into an aluminum gas bag containing 30 L of nitrogen, water and methanol were added using a syringe to achieve a water concentration of 15,000 ppmv and a methanol concentration of 5,000 ppmv. Gas from the aluminum gas bag was passed through a column with an inner diameter of 6 mm filled with 50 mg of sample, at a temperature of 25°C and a flow rate of 50 mL/min. Gas exiting the column was analyzed using an FTIR spectrometer (JASCO Corporation, FT/IR-6600, hereinafter the same).

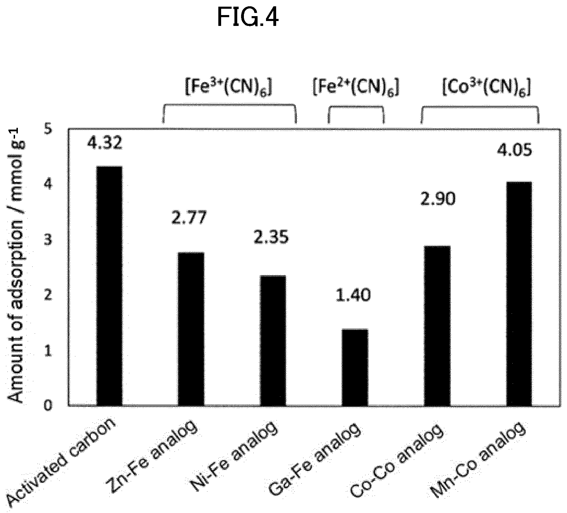

The methanol adsorption capacity was calculated by subtracting the cumulative amount of methanol detected by FTIR from the initial amount of methanol in the aluminum gas bag. The methanol adsorption capacities of each sample are shown in Figure 4. As shown in Figure 4, all samples adsorbed methanol from the gas containing water. On the other hand, the methanol adsorption capacity of the Prussian blue analogs from Production Examples 1 to 5 was found to depend on the type and valence of M' bonded on the carbon side in the structure AₓM[M'(CN)₆]y·zH₂O of the Prussian blue analogs. The order of methanol adsorption capacities for the Prussian blue analogs was as follows: Mn-Co analog or Co-Co analog (M' = Co³⁺) > Ni-Fe analog or Zn-Fe analog (M' = Fe³⁺) > Ga-Fe analog (M' = Fe²⁺).

### Example 2: Evaluation of Methanol Adsorption Energy

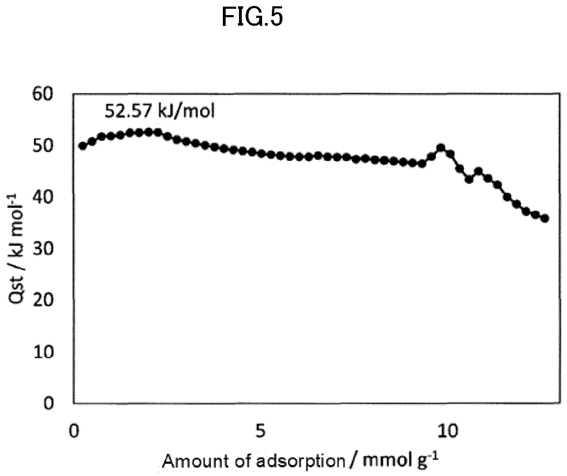

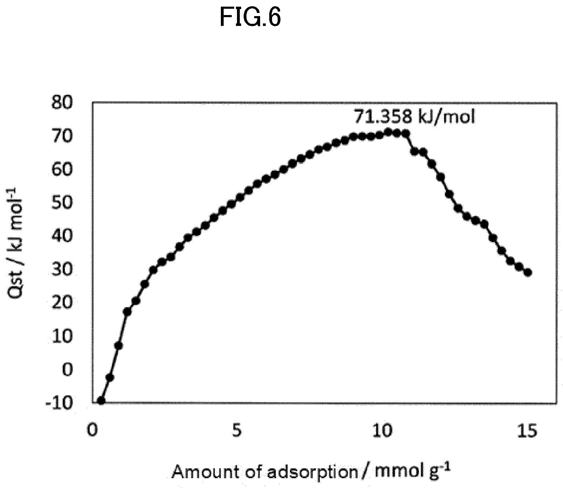

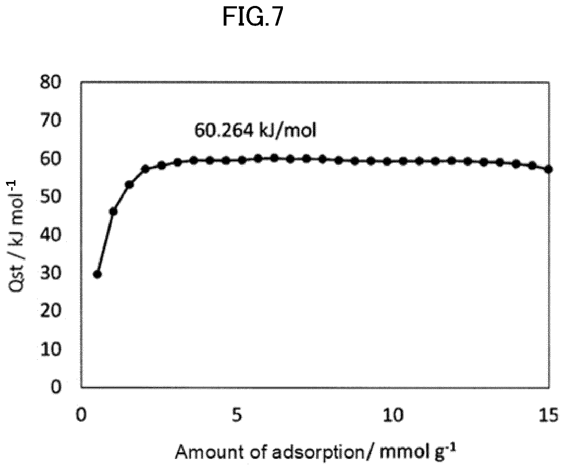

Using a specific surface area/pore distribution measurement device (BEL Japan, Inc., Belsorp-MAX II), methanol adsorption isotherms at 25°C and 40°C for activated carbon and Mn-Co analog, as well as water adsorption isotherms at 25°C and 40°C for Mn-Co analog, were created. The methanol adsorption energy was calculated from the methanol adsorption isotherms at two temperatures using analysis software (BEL Japan, Inc., Belmaster) based on the Clausius-Clapeyron equation. The methanol differential adsorption heat curve for activated carbon is shown in Figure 5, and the methanol differential adsorption heat curve for Mn-Co analog is shown in Figure 6. Similarly, the water adsorption energy was calculated from the water adsorption isotherms at two temperatures. The water differential adsorption heat curve for Mn-Co analog is shown in Figure 7.

As shown in Figure 5, the maximum methanol adsorption energy of activated carbon was 52.5 kJ/mol. On the other hand, as shown in Figure 6, the maximum methanol adsorption energy of the Mn-Co analog was 71.4 kJ/mol, indicating a high value. This shows that the Mn-Co analog is more likely to adsorb methanol and less likely to desorb methanol compared to activated carbon. Furthermore, as shown in Figure 7, the maximum water adsorption energy of the Mn-Co analog was 60.3 kJ/mol. This shows that the Mn-Co analog is more likely to adsorb methanol than water and less likely to desorb methanol than water.

### Example 3: Evaluation of Methanol Desorption Temperature

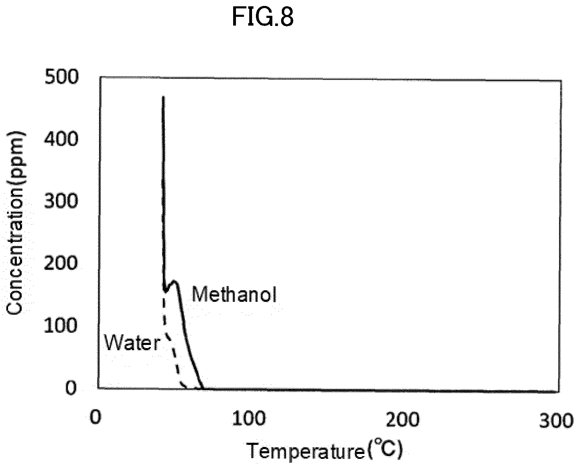

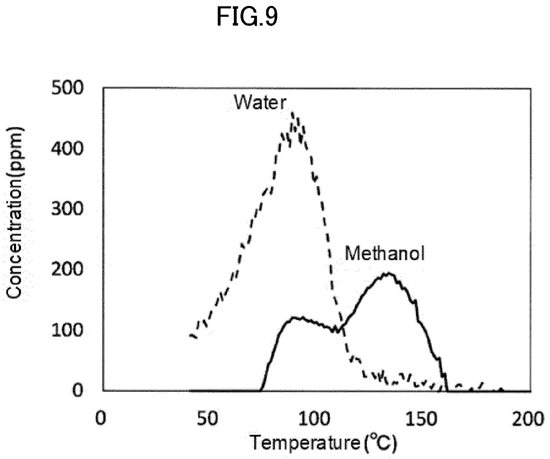

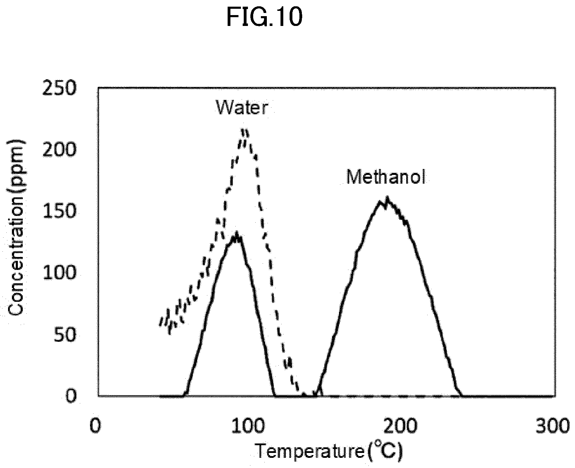

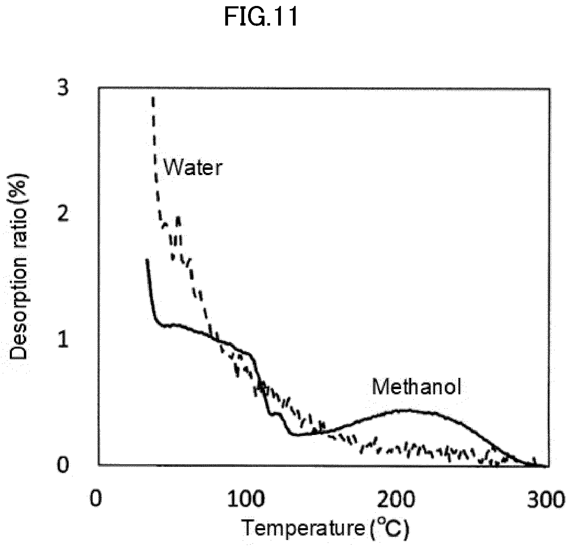

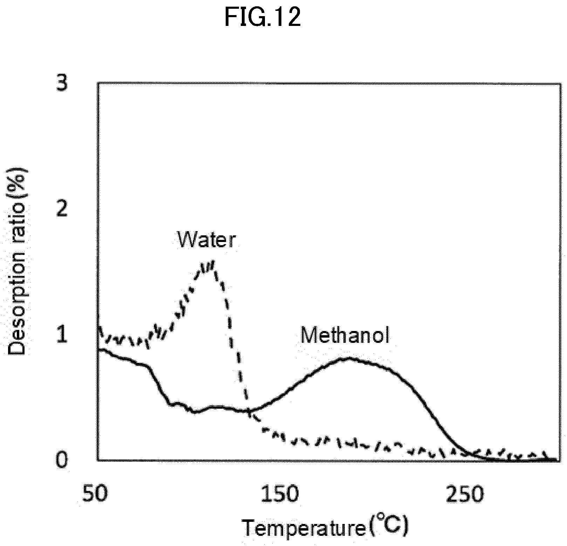

Air containing a water concentration of 15,000 ppmv and a methanol concentration of 5,000 ppmv was placed in five containers. In each container, one of five samples-activated carbon and the Prussian blue analogs from Production Examples 1 to 4-was placed. The containers were left undisturbed at approximately 20-30°C for three days to allow adsorption of water and methanol. Using TG-MS (JEOL, JMS-Q1500GC) under conditions of a helium flow rate of 211 mL/min and a temperature increase rate of 5°C/min, the desorption of water and methanol from these samples was measured. Figure 8 shows the TG-MS curve of activated carbon. Figure 9 shows the TG-MS curve of the Mn-Co analog. Figure 10 shows the TG-MS curve of the Co-Co analog. Figure 11 shows the TG-MS curve of the Ga-Fe analog. Figure 12 shows the TG-MS curve of the Ni-Fe analog.

In Figures 8 to 10, desorption was considered complete when the weight change of the sample became 0.03 wt%/°C or less, and it was assumed that the mass of water and methanol adsorbed on the sample matched the mass of water and methanol desorbed from the sample. The intensity of m/z = 31 detected by MS was converted to methanol concentration, and the intensity of m/z = 19 was converted to water concentration, with the results displayed as curves. In Figures 11 and 12, it was assumed that all water and methanol adsorbed on the sample desorbed (100% desorption rate) at 300°C, and the desorption rates of water and methanol at each measurement temperature were displayed as curves.

As shown in Figure 8, in activated carbon, the desorption temperatures of water and methanol overlapped, making it difficult to selectively desorb methanol alone. On the other hand, as shown in Figures 9 and 10, in the Co-Co analog and Mn-Co analog, some methanol desorbed together with water below 100°C, but almost exclusively methanol desorbed at temperatures of 120°C or higher. Thus, by recovering the gas desorbed from the Co-Co analog or Mn-Co analog at 120°C or higher, and cooling it, high-concentration methanol can be obtained. These findings indicate that Prussian blue-type complexes have superior separation capabilities for water and methanol compared to activated carbon.

As shown in Figures 11 and 12, in the Ga-Fe analog and Ni-Fe analog, similar to the Mn-Co analog and Co-Co analog, high-concentration methanol containing little water desorbed at temperatures of 120°C or higher.

From the above, it was found that by heating the Prussian blue analogs (Production Examples 1 to 5) that had adsorbed water and methanol to 120°C or higher (the second temperature), recovering the desorbed gas, and cooling it, high-concentration methanol can be obtained.

### Example 4: Evaluation of Methanol Adsorption from Methanol Aqueous Solution

In 1 mL of 5 vol% methanol aqueous solution, 0.2 g of activated carbon or the Mn-Co analog from Production Example 1 was added and shaken for 120 minutes. The supernatant after shaking was evaluated using the FTIR-ATR method, and the methanol concentration in the aqueous solution was calculated from the absorption peak at 1014 cm⁻¹. The methanol adsorption capacity was calculated by subtracting the amount of methanol detected in the supernatant after shaking from the initial amount of methanol in the aqueous solution prior to adding the activated carbon or Mn-Co analog. The methanol adsorption capacity of activated carbon was 32.6 mg/g, whereas the methanol adsorption capacity of the Mn-Co analog was 69.4 mg/g.
It was determined that the Mn-Co analog is effective for recovering methanol from water.

### Example 5: Evaluation of Methanol Concentration

A column with an inner diameter of 10 mm, filled with 400 mg of the Mn-Co analog from Production Example 1, was used. Nitrogen gas containing 15,000 ppmv water vapor and 5,000 ppmv methanol was passed through the column at 25°C and a flow rate of 200 mL/min for 150 minutes to allow the Mn-Co analog to adsorb water and methanol (adsorption step). Subsequently, nitrogen gas was passed through the column at 70°C and a flow rate of 200 mL/min for 180 minutes (dehydration step), followed by nitrogen gas at 150°C and a flow rate of 100 mL/min for 180 minutes (methanol desorption step). Water and methanol were desorbed, and the concentrations of water and methanol in the outlet gas were evaluated using FTIR.

The amounts of water and methanol desorbed from the Mn-Co analog at 150°C were calculated by integrating the gas concentrations detected over time by FTIR. Assuming that all the water and methanol were recovered at liquid nitrogen temperature, the methanol concentration (methanol mass / (methanol mass + water mass) × 100, hereinafter the same) was 92%. From these results, it was confirmed that methanol can be recovered from humid air containing methanol using the Mn-Co analog. Furthermore, by introducing a water desorption step where the Mn-Co analog with adsorbed water and methanol is maintained at a temperature below 120°C (the first temperature), it was demonstrated that high-concentration methanol can be recovered in the subsequent methanol desorption step, where the Mn-Co analog is maintained at 120°C or higher (the second temperature).

### Example 6: Durability Evaluation

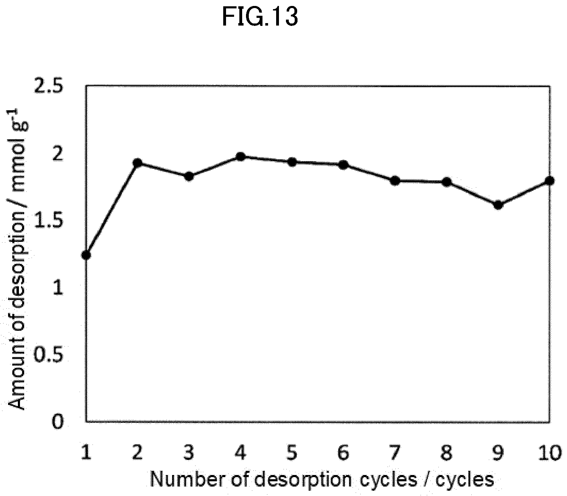

Under the same conditions as in Example 5, the process consisting of the adsorption step, water desorption step, and methanol desorption step was repeated 10 times, and the amount of desorbed methanol was evaluated using FTIR. The amount of methanol desorbed from the Mn-Co analog at 150°C is shown in Figure 13. As shown in Figure 13, even after repeating the adsorption and desorption processes 10 times, the Mn-Co analog was able to adsorb methanol, demonstrating that high-concentration methanol can still be recovered from the Mn-Co analog.

### Example 7: Evaluation of Methanol Selective Adsorption

A column with an inner diameter of 10 mm, filled with 400 mg of the Mn-Co analog from Production Example 1, was maintained at 70°C while nitrogen gas containing 15,000 ppmv water vapor and 5,000 ppmv methanol was passed through the column at a flow rate of 200 mL/min for 150 minutes. As in Example 3, the amounts of water and methanol adsorbed onto the Mn-Co analog were calculated. The results showed that 24 mg/g of methanol was adsorbed onto the Mn-Co analog, but no water was adsorbed. These findings indicate that there exists a temperature range in which the Mn-Co analog adsorbs methanol without adsorbing significant amounts of water.

In other words, by heating the Prussian blue analog of the present invention to a specific temperature range, such as approximately 70°C, and passing a gas containing water and methanol through it, methanol can be selectively adsorbed. This temperature range can be identified without excessive trial and error using the method described in this example. Furthermore, by heating the Prussian blue analog, which has selectively adsorbed methanol, to a higher temperature, such as approximately 120°C, and recovering and cooling the desorbed gas, high-concentration methanol can be obtained.

### Example 8: Examination of the Third Temperature

Methanol was concentrated using the same method as in Example 5, except that the water desorption step at 70°C was omitted. The methanol concentration in the gas at the outlet of the column after concentration was 42%. This corresponds to the case in the high-concentration methanol production method of the second embodiment, where the third temperature is 25°C and the fourth temperature is 150°C. On the other hand, the results of Example 5 correspond to the case in the high-concentration methanol production method of the second embodiment, where the third temperature is 70°C and the fourth temperature is 150°C. In this case, the methanol concentration of the high-concentration methanol obtained is expected to be 92%. Assuming that the methanol concentration after concentration is proportional to the absolute temperature of water and methanol adsorption (the third temperature), it can be calculated that a methanol concentration of 54%, suitable for use in direct methanol fuel cells, would be achieved at a third temperature of 35°C.

### Example 9: Methanol Adsorption Test in Liquid

As a comparison to the Mn-Fe analog (Production Example 6), activated carbon and zeolite powders were added to centrifuge tubes. For each sample, 24 tubes × 3 types were prepared. To each centrifuge tube, one of eight methanol solutions (n = 3) adjusted to methanol concentrations of 50,000, 20,000, 10,000, 5,000, 2,000, 1,000, 500, and 100 mg-methanol/L was added.

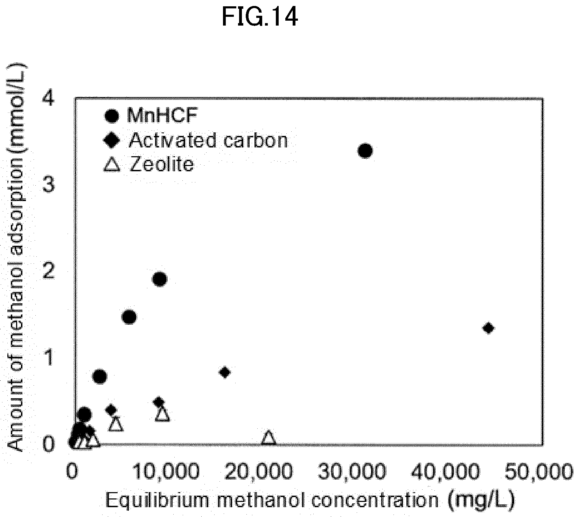

The samples were shaken at 25°C for more than 24 hours until the adsorbents adsorbed methanol and the liquid methanol concentration reached equilibrium. After shaking, the liquid was collected, and the equilibrium methanol concentration and methanol adsorption capacity were determined by measuring the liquid with GC/MS. The results are shown in Figure 14. As shown in Figure 14, it was found that the Mn-Fe analog (MnHCF in Figure 14) can adsorb methanol even at low equilibrium methanol concentrations.

### Example 10: Comparison of Methanol Adsorption Capacity at 1000 mg-Methanol/L Equilibrium

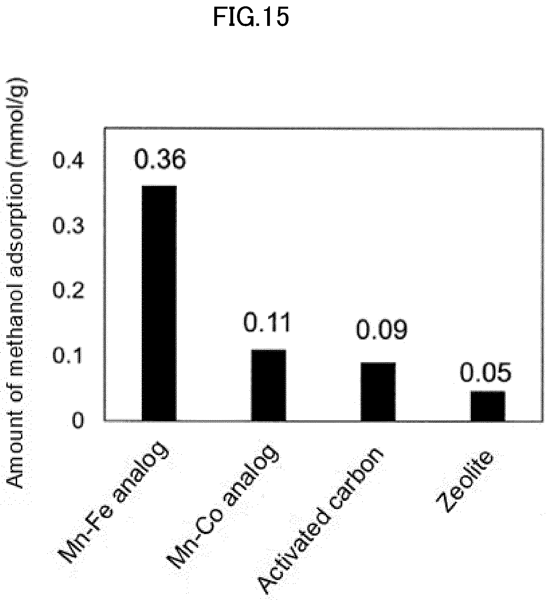

As a comparison to the Mn-Fe analog (Production Example 6), Mn-Co analog (Production Example 1) powder was packed into separate columns. Methanol water with a concentration of 1000 mg-methanol/L was passed through the columns at 25°C. When the methanol adsorption capacities of each sample were compared, the results were:
Mn-Fe analog: 0.36 mmol/g
Mn-Co analog: 0.11 mmol/g (Figure 15).
Additionally, in Figure 15, this result is compared with the methanol adsorption capacities at 1000 mg-methanol/L equilibrium for activated carbon and zeolite, calculated by interpolation from their adsorption isotherms.

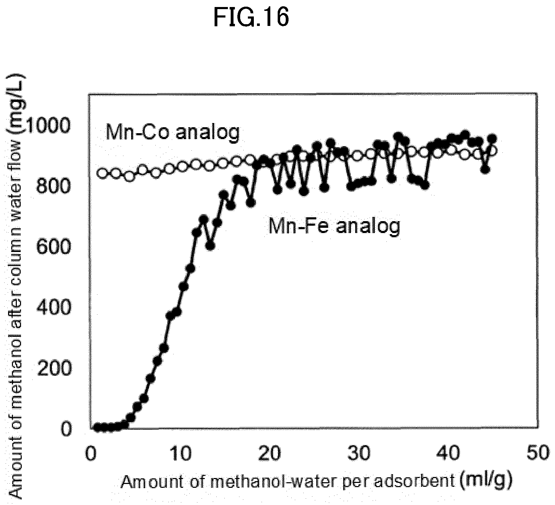

The results of Example 10 are shown in Figure 16. In Figure 16, the methanol concentration after passing through the column is plotted against the flow rate of methanol water with a concentration of 1000 mg-methanol/L per gram of adsorbent packed into the column. While the Mn-Co analog showed almost no adsorption from the beginning, the Mn-Fe analog adsorbed methanol, resulting in the initial solution after passing through the column containing almost no methanol. As the flow rate of methanol water passing through the column increased, the Mn-Fe analog's methanol adsorption capacity decreased, eventually reaching equilibrium with the concentration before passing through the column.

### [Explanation of Symbols]

10 Methanol recovery device
12 Housing
14 Heating part
16 Holding part
18 First supply part
20 Second supply part
22 Recovery part
24 Discharge part

## Claims

1. A methanol adsorbent for adsorbing methanol contained in a liquid or gas comprising water and methanol,
wherein the methanol adsorbent comprises, as an active component, a Prussian blue analog represented by the following general formula (1):
AₓM[M'(CN)₆]y·zH₂O (1)
(wherein x, y, and z satisfy 0 ≦ x ≦ 3, 0.1 ≦ y ≦ 1.5, and 0 ≦ z ≦ 6; A represents one or more of hydrogen ions, ammonium cations, alkali metal ions, and alkaline earth metal ions; and M and M' are cations independent of each other, excluding hydrogen ions, ammonium cations, alkali metal ions, and alkaline earth metal ions.)

2. The methanol adsorbent according to claim 1,
wherein M is any one of Mn²⁺, Co²⁺, Ga²⁺, Ni²⁺, or Zn²⁺, and M' is Co³⁺ or Fe³⁺.

3. The methanol adsorbent according to claim 1,
wherein M is Mn²⁺, and M' is Co³⁺ or Fe³⁺.

4. A method for producing high-concentration methanol, comprising:
an adsorption step of bringing the methanol adsorbent according to any one of claims 1 to 3 into contact with a liquid or gas comprising water and methanol to adsorb water and methanol onto the methanol adsorbent;
a water desorption step of maintaining the methanol adsorbent, after the adsorption step, at a first temperature to desorb water from the methanol adsorbent; and
a methanol desorption step of maintaining the methanol adsorbent, after the water desorption step, at a second temperature higher than the first temperature to desorb and recover high-concentration methanol from the methanol adsorbent.

5. The method for producing high-concentration methanol according to claim 4,
wherein the first temperature is 70°C or higher and less than 120°C, and the second temperature is 120°C or higher and 250°C or lower.

6. A method for producing high-concentration methanol, comprising:
a methanol adsorption step of bringing the methanol adsorbent according to any one of claims 1 to 3 into contact with a liquid or gas comprising water and methanol at a third temperature to selectively adsorb methanol onto the methanol adsorbent over water; and
a methanol recovery step of maintaining the methanol adsorbent, after the methanol adsorption step, at a fourth temperature higher than the third temperature to desorb and recover high-concentration methanol from the methanol adsorbent.

7. The method for producing high-concentration methanol according to claim 6, wherein the third temperature is 35°C or higher and less than 120°C, and the fourth temperature is 120°C or higher and 250°C or lower.

8. A methanol recovery device comprising:
a methanol adsorbent according to any one of claims 1 to 3;
a holding part for retaining the methanol adsorbent therein;
a heating part capable of heating the methanol adsorbent in the holding part to a temperature of 35°C or higher and 250°C or lower;
a first supply part for supplying a liquid or gas comprising water and methanol to the methanol adsorbent in the holding part; and
a recovery part for recovering high-concentration methanol desorbed from the methanol adsorbent in the holding part.
